(19) Europäisches Patentamt
European Patent Office
Office européen des brevets

(11) Numéro de publication: 0 269 517 B1

(12) # FASCICULE DE BREVET EUROPEEN

(45) Date de publication du fascicule du brevet: **07.11.90**

(51) Int. Cl.[5]: **C07C 319/24**, C07C 321/14, B01J 31/26

(21) Numéro de dépôt: **87402620.6**

(22) Date de dépôt: **19.11.87**

(54) **Procédé de production de polysulfures organiques et système catalytique pour sa réalisation.**

(30) Priorité: **28.11.86 FR 8616615**

(43) Date de publication de la demande: **01.06.88 Bulletin 88/22**

(45) Mention de la délivrance du brevet: **07.11.90 Bulletin 90/45**

(84) Etats contractants désignés: **BE DE FR GB IT NL**

(56) Documents cités: **EP-A- 0 025 944**

(73) Titulaire: **SOCIETE NATIONALE ELF AQUITAINE (PRODUCTION), Tour Elf 2, Place de la Coupole La Défense 6, F-92400 Courbevoie(FR)**

(72) Inventeur: **Gongora, Henri, 5 Parc Résidence Chemin Salié, F-64050 Lons Billère(FR)**
Inventeur: **Darche, Yves, 15 Lotissement Bourdette, F-64300 Orthez(FR)**

(74) Mandataire: **Leboulenger, Jean et al, ATOCHEM Département Propriété Industrielle, F-92091 Paris la Défense 10 Cédex 42(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention se rapporte à la fabrication de polysulfures organiques par l'action du soufre sur un mercaptan, en milieu liquide, en présence d'un nouveau système catalytique.

Des polysulfures organiques sont des produits ayant diverses applications industrielles, en particulier dans les huiles de coupe, comme additifs extrême pression. Leur préparation en milieu liquide, par réaction entre le soufre élémentaire et un mercaptan, est connue. Elle exige en général la présence d'un catalyseur, comme amine, alcanolamine, base inorganique, mercaptide ou alcoolate. De tels procédés ont été décrits par exemple dans le brevet français n° 1 381 265 et dans les brevets US n° 3 275 693 ; 3 314 999 et 3 340 324. On constate cependant que ces dif férents catalyseurs comportent certains inconvénients : ou bien le rendement laisse à désirer, ou la pureté du produit n'est pas satisfaisante ou encore le polysulfure obtenu dégage une odeur gênante. Ainsi, les amines confèrent-elles une odeur et une certaine turbidité ; les mercaptides et alcoolates exigent l'emploi d'un solvant qui cause la turbidité du produit fabriqué et augmente les frais de fabrication.

La présente invention, grâce à l'utilisation d'un nouveau catalyseur, spécial, évite les inconvénients précités et permet la production régulière, en continu, sans solvant, avec de très bons rendements, de polysulfures de bonne pureté, sans odeur gênante, sans turbidité ni couleur indésirable et avec un très faible taux de -SH residuel. Elle s'applique à de nombreux polysulfures du type $R-S_n-R$ où R est un groupe hydrocarboné, notamment aliphatique, cycloaliphatique ou arylique, n moyen pouvant varier entre 2 et 8 et surtout 3 et 5. Les polysulfures les plus employés actuellement étant ceux dont les groupes R sont des alkyles linéaires ou ramifiés, le procédé de l'invention permet de les obtenir aisément. Ainsi peut-on produire des polysulfures avec, comme R, des alkyles en $C_1$ à $C_{20}$ et, en particulier $C_6$ à $C_{18}$, parmi lesquels comptent les composés fort utiles, à alkyles tertiaires, notamment en $C_8$ à $C_{16}$, plus spécialement ter-nonyl- et ter-dodécylpolysulfures, dont le n est de 3 à 5.

Le procédé suivant l'invention, qui consiste à chauffer un ou plusieurs mercaptans à l'état liquide, avec du soufre, en présence d'un catalyseur, est caractérisé en ce que le catalyseur est constitué par la combinaison d'un mercaptan avec un oxyde d'alcène et une base alcaline. Conviennent particulièrement les alcènes en $C_2$ à $C_4$.

La combinaison, servant de catalyseur selon l'invention, peut être représentée par l'expression

$RSH.x(C_mH_{2m}O).yMOH$

où R désigne un groupe hydrocarboné, en particulier alkyle, comme ceux dont il est question plus haut ; x est le nombre de moles d'oxyde d'alcène par mole de mercaptan RSH, et y celui des moles de base alcaline, M désignant un métal alcalin, surtout Na ou K. Comme oxyde d'alcène, on utilise de préférence l'oxyde d'éthylène plus économique, mais il peut éventuellement être remplacé par ou mélangé à un autre oxyde d'alcène.

En général, x a une valeur de 1 à 20 et le plus souvent de 4 à 12 ; elle dépend du caractère tensio-actif requis pour la synthèse du polysulfure, de la compatibilité du catalyseur avec le polysulfure à fabriquer et de l'homogénéité du système catalytique.

Quand à y, il est de préférence d'environ 0,01 à 1, ou mieux de 0,1 à 0,5.

Le système catalytique, décrit plus haut, est employé tel quel, pour être ajouté à la masse de mercaptan que l'on veut transformer en polysulfure, ou bien à l'état dilué avec une portion de ce mercaptan. Bien que la proportion de catalyseur par rapport au mercaptan à transformer puisse varier assez largement, elle est de préférence d'environ 0,1 à 5 % en poids.

Un mode de préparation du nouveau système catalytique consiste à mélanger d'abord 1 mole d'un mercaptan RSH avec $\alpha y$ moles ($\alpha$ = 1 à 1,5) de base alcaline MOH, de préférence en homogénéisant ce mélange à chaud ; on peut notamment opérer entre 50° et 100°C pendant plusieurs heures, en particulier entre 75° et 85°C durant 4 à 6 heures. Dans le liquide ainsi obtenu, on injecte ensuite de l'oxyde d'alcène de façon à fixer la proportion voulue x, indiquée plus haut, de groupes $C_mH_{2m}O$. Cette injection s'effectue de préférence sous une pression de 0,2 à 4 bars à une température de l'ordre de 80° à 120°C selon la nature du mercaptan traité ; la fixation de l'oxyde d'alcène dure en général 1 à 10 heures.

Après ces opérations, il est recommandable de dégazer le milieu réactionnel décomprimé, afin d'éliminer l'excès d'oxyde dissous ; le dégazage se fait avantageusement à l'aide d'un gaz inerte, tel qu'azote.

Il est bon de filtrer le liquide obtenu, avant de l'utiliser à la fabrication du polysulfure.

Une installation, pour la préparation du système catalytique, comprend essentiellement un réacteur où a lieu la fixation de l'oxyde d'alcène sur le mercaptan alcalinisé, ainsi qu'une cuve de dégazage de l'oxyde excédentaire, surmontée de colonnes appropriées. Ces appareils et leurs accessoires peuvent s'intégrer avantageusement dans une installation de polysulfuration d'un mercaptan, de façon à fournir à celle-ci le catalyseur nécessaire.

Le procédé suivant l'invention, utilisant le nouveau catalyseur, peut être - d'une façon générale - conduit comme les procédés connus, appliquant l'action du soufre sur un ou plusieurs mercaptans à l'état liquide. Lorsque le mercaptan utilisé comporte plus de 6 atomes de carbone, il est indiqué d'effectuer la polysulfuration à une température de l'ordre de 60° à 150°C et, en particulier, entre 70° et 140°C d'autant plus basse que le rang en soufre, voulu pour le polysulfure, est élevé. Ainsi, par exemple, à partir du ter dodécyl-mercaptan on peut obtenir le rang 5, soit $RS_5R$, à 75°C, et le rang 3, c'est-à-dire $RS_3R$, à 130°C.

Les pressions relatives, préférées, se rangent entre 0,4 et 1 bar.

La réaction étant :

$2RSH + (n\text{-}1)S \rightarrow R\text{-}S_n\text{-}R + H_2S$

le rapport molaire S/RSH, nécessaire à l'obtention d'un polysulfure de rang n en soufre, se calcule comme (n-1):2.

Le procédé de l'invention se prête particulièrement bien au travail en continu,les trois matières de départ, mercaptan, soufre et catalyseur, pouvant être introduites simultanément à l'état liquide dans un réacteur. Dans ce mode opératoire, le produit brut, obtenu dans un premier réacteur, subit un dégazage, notamment par soufflage de l'azote, dans un appareil approprié, avant d'être acheminé dans un second réacteur où la réaction est terminée.

Après filtration, on recueille un polysulfure qui ne contient plus de catalyseur.

Dans la fabrication continue, l'alimentation du réacteur en catalyseur peut provenir d'une production également continue de ce catalyseur. Une installation pour la préparation de celui-ci est alors associée aux appareils de production de polysulfure.

L'invention est illustrée par les exemples non limitatifs, qui suivent.

EXEMPLE 1

Préparation du nouveau catalyseur

Dans un réacteur de 150 litres, en acier inoxydable, on introduit 68 kg (0,3366 Kmole) de ter-dodécylmercaptan $C_{12}H_{25}SH$, avec 6,3 kg de NaOH anhydre (0,1575 Kmole). On assure une bonne dispersion et homogénéisation du mélange par chauffage à 80°C pendant 5 heures. L'oxyde d'éthylène est alors injecté dans le réacteur avec un débit continu de 7 kg/h ; la pression relative dans le reacteur passe de 0,2 bar au début à 4 bars à la fin de la réaction.

On chauffe ensuite le milieu réactionnel à 100° -110°C pendant 2 heures, puis on le décomprime et on le dégaze par passage de l'azote, de façon à éliminer tout l'oxyde d'éthylène excédentaire, dissous. Le liquide obtenu est soumis à la filtration qui arrête les particules solides jusqu'à 5mμ. Le système catalytique résultant présente la composition :

$$[C_{12}H_{25}S(CH_2CH_2O)_6H]\ (NaOH)_{0,349}$$

c'est-à-dire :

$$[C_{12}H_{25}S(CH_2CH_2O)_6H]\ \ 3\%\ NaOH$$

soit, en poids :

$C_{12}H_{25}SH$ .................. 42%
$CH_2CH_2O$ .................... 55%
NaOH ...................... 3%

Le point de trouble de ce catalyseur est à 62°C environ.

EXEMPLE 2

Production de trisulfure de di(tert.nonyle) $(C_9H_{19})_2S_3$.

En opérant avec deux réacteurs successifs, comme exposé plus haut, on utilise le tert.nonyl-mercaptan et le soufre fondu à raison de 1 atome S pour 1 mole $C_9H_{19}SH$, soit 20 kg S pour 100 kg de mercaptan. On ajoute à 100 kg de mercaptan 1,25 kg de catalyseur $C_9H_{19}S(CH_2CH_2O)_6H.(NaOH)_{0,349}$ préparé de façon analogue à celle qu'indique l'exemple 1. Le mélange est chauffé, dans le premier réacteur, à 130°C, pendant 1h15 et soumis au dégazage par soufflage de 18 Nm³ d'azote par 100 kg de mercaptan initial.

Ensuite a lieu le chauffage terminal, dans le second réacteur, à 130°C, pendant 2h30, et l'on effectue le dégazage à l'aide de 18 Nm³ de $N_2$ par 100 kg de mercaptan mis en oeuvre.

Lorsque cette fabrication est menée en continu, on peut obtenir 800 kg de polysulfure par heure,par m³ de capacité du réacteur principal de synthèse.

EXEMPLE 3

Préparation de trisulfure de di-(tert.dodécyle) $(C_{12}H_{25})_2S_3$.

On opère de la même façon qu'à l'exemple 2, mais en partant de tert.dodécyl-mercaptan $C_{12}H_{25}SH$ additionné de 1,25% de catalyseur obtenu selon l'exemple 1. La proportion de soufre fondu est de 15,84 kg pour 100 kg de mercaptan, toutes les autres conditions opératoires étant les mêmes que dans l'exemple 2. Le polysulfure est obtenu avec un rendement de 98% sur le mercaptan mis en oeuvre, et la productivité est d'environ 800 kg/h par m$^3$ de réacteur, lorsqu'on opère en continu.

EXEMPLE 4

Production de pentasulfure de di-tert.dodécyle $(C_{12}H_5)_2S_5$

On opère, comme plus haut, avec deux réacteurs successifs, mais à la même température de 75°C dans chacun d'eux. Les dégazages ont lieu également à 75°C, chacun avec 18 Nm$^3$ d'azote par 100 kg de mercaptan. La quantité de soufre utilisé est de 2 atomes par mole de tert-dodécyl-mercaptan de départ, soit 31,9 kg S pour 100 kg $C_{12}H_{25}SH$. Le catalyseur de l'exemple 1 est ajouté à raison de 0,4 kg par 100 kg de mercaptan.
La pression relative varie entre 0,4 et 1 bar.
Dans l'exécution du procédé en continu, la productivité atteint 1000 kg de polysulfure par heure,par m$^3$ de réacteur principal de synthèse.

EXEMPLE 5

Production de pentasulfure de di-tert.dodécyle en continu.

Un premier réacteur, de 150 l de capacité, est alimenté en continu avec 80 kg de ter.dodécyl-mercaptan à l'heure, de 0,32 kg/h de catalyseur suivant l'exemple 1, et avec 22,8 kg/h de soufre liquide. Le contenu du réacteur est maintenu à 75°C, et il est vigoureusement agité par circulation, au moyen d'une pompe, dans une boucle externe au réacteur. L'$H_2S$ formé est éliminé à la torche, alors que le liquide sortant du réacteur subit un dégazage à l'azote dans une colonne prévue à cet effet, avec 5 Nm$^3$/h d'azote. Ce liquide passe dans le second réacteur où il est encore chauffé à 75°C, puis dégazé avec 10 Nm$^3$/h d'azote. Après filtration à 60°C, on a 95 kg de produit fini (Rendement 98%), dont la teneur en SH résiduel est inférieure à 10 ppm, celle de soufre est de 31,5% et la couleur $\leq$10 Gardner.
Ce produit ne présente aucune odeur indésirable ni turbidité.

EXEMPLE 6

Comparatif

Les opérations de l'exemple 4 sont répétées, mais avec remplacement du catalyseur oxyéthyléné par de la triéthylamine. Même après le dégazage, opéré comme dans l'exemple 4, le polysulfure obtenu présente une odeur désagréable, une couleur et une turbidité marquées, alors que celui de l'exemple 4, suivant l'invention, n'a plus d'odeur désagréable et est clarifié. De plus, la basicité renforcée du nouveau catalyseur facilite une conversion plus rapide et plus complète du mercaptan.

EXEMPLE 7

Comparatif

On répète les opérations de l'exemple 3, en remplaçant le catalyseur, suivant l'invention, par 0,125 moles de NaOH et 0,125 moles d'hexanol par mole de tert.dodécyl-mercaptan, comme indiqué dans le brevet US 3 340 324. Le polysulfure n'est obtenu qu'avec un rendement de 70% par rapport au mercaptan utilisé, au lieu de 98% dans l'exemple 3, et il est fortement turbide alors que celui de l'exemple 3 est parfaitement homogène.

EXEMPLE 8

Dans l'exemple 1, on remplace le catalyseur suivant l'invention par la même quantité de mercaptide de sodium correspondant. Le milieu réactionnel est alors hétérogène et le produit obtenu est turbide.

Ces mêmes inconvénients sont constatés, lorsque le catalyseur employé est l'éthanolate de sodium.

En définitive le procédé suivant l'invention apporte l'avantage de permettre l'obtention de polysulfure à rang moyen, bien déterminé, en soufre, grâce à l'alimentation en continu des réactifs, à savoir du soufre et du mercaptan, avec la stoechiométrie adéquate. Cela n'est pas réalisé en discontinu, où le défaut de soufre, pendant son alimentation dans la charge de mercaptan, conduit à la formation de polysulfures

de rangs inférieurs, non désirables. Comme déjà vu plus haut, l'invention fait disparaitre la turbidité des produits finis, due au soufre dissous, qui n'a pas réagi. Le nouveau procédé apporte donc une meilleure régularité de la fabrication et de la qualité des produits finis.

## Revendications

1. Procédé de production de polysulfures organiques du type $R-S_n-R$, où R est un groupe hydrocarboné et n un nombre de 2 à 8,par chauffage d'un ou de plusieurs mercaptans avec du soufre, en présence d'un catalyseur, caractérisé en ce que le catalyseur est constitué par la combinaison d'un mercaptan avec un oxyde d'alcène et une base alcaline.

2. Procédé suivant la revendication 1, caractérisé en ce que le catalyseur est une combinaison $RSH.x(C_mH_{2m}O).yMOH$ où R désigne un groupe hydrocarboné, en particulier un alkyle, x est le nombre de moles d'oxyde d'alcène par mole de mercaptan, M désigne un atome de métal alcalin et y le nombre de moles de base alcaline par mole de mercaptan.

3. Procédé suivant la revendication 1 ou 2, caractérisé en ce que x est 1 à 20, et de préférence 4 à 12, tandis que y est 0,01 à 1, de préférence 0,1 à 0,5.

4. Procédé suivant une des revendications précédentes, caractérisé en ce que R est un alkyle en $C_1$ à $C_{20}$, et, en particulier, en $C_6$ à $C_{18}$, l'oxyde d'alcène étant en $C_2$ à $C_4$ et de préférence l'oxyde d'éthylène.

5. Procédé suivant la revendication 4, caractérisé en ce que l'alkyle est ramifié, notamment un alkyle tertiaire, et en particulier tert.nonyle ou tert.dodécyle.

6. Procédé suivant une des revendications précédentes, caractérisé en ce que le chauffage a lieu à une temprature de 60° à 150°C, plus particulièrement entre 70° et 140°C

7. Procédé suivant la revendication 6, dans lequel n a une valeur de 3 à 5, caractérisé en ce que l'on opère à une température d'autant plus élevée que le nombre n désiré est bas.

8. Procédé suivant une des revendications précédentes, caractérisé en ce que le chauffage du mercaptan avec le soufre et le catalyseur est effectué, en deux étapes, chacune étant suivie d'un dégazage de l'$H_2S$, notamment au moyen d'un gaz inerte.

9. Procédé suivant une des revendications précédentes, caractérisé en ce qu'il est réalisé sous une pression relative de 0,2 à 1 bar.

10. Procédé suivant une des revendications précédentes, caractérisé en ce que le catalyseur est préparé par homogénéisation d'une mole d'un mercaptan avec $\alpha y$ moles ($\alpha = 1$ à 1,5) de base alcaline, de préférence entre 50° et 100°C, puis saturation du produit obtenu avec de l'oxyde d'alcène à une température de 80° à 120°C sous une pression relative de 0,2 à 4 bars, suivie d'un dégazage de l'oxyde d'alcène excédentaire, de préférence au moyen d'un gaz inerte.

11. Procédé suivant la revendication 10, dans lequel le mercaptan de départ est en $C_6$ à $C_{18}$, en particulier un mercaptan de tert.alkyle, caractérisé en ce que l'homogénéisation a lieu entre environ 75° et 85°C durant 4 à 6 heures et la saturation avec l'oxyde d'alcène à environ 100 à 110°C.

12. Procédé suivant une des revendications précédentes, réalisé en continu.

13. Nouveau système catalytique, caractérisé en ce qu'il comprend une combinaison $R-S(C_mH_{2m}O)_xH.(MOH)_y$ dans laquelle R est un alkyle en $C_1$ à $C_{20}$, x un nombre de 1 à 20, m un nombre de 2 à 4, M est un métal alcalin et y un nombre de 0,01 à 1.

14. Système catalytique selon la revendication 13, dans lequel R est un groupe alkyle en $C_6$ à $C_{18}$, m est égal à 2 et x est un nombre de 4 à 12 et y un nombre de 0,1 à 0,5.

## Patentansprüche

1. Verfahren zur Herstellung von organischen Polysulfiden des Typs $R-S_n-R$, worin R eine kohlenwasserstoffhaltige Gruppe und n eine Zahl von 2 bis 8 ist, durch Erhitzen eines oder mehrerer Mercaptane mit Schwefel in Gegenwart eines Katalysators, dadurch gekennzeichnet, daß der Katalysator aus der Zusammensetzung eines Mercaptans mit einem Alkenoxid und einer Alkalibase gebildet wird.

2. Verfahren nach Anspruch 1, dadurch gekennzeichnet, daß der Katalysator eine Zusammensetzung der Formel $RSH.x(C_mH_{2m}O).yMOH$ ist, worin R eine kohlenwassermstoffhaltige Gruppe, insbesondere ein Alkyl bezeichnet, x die Molzahl des Alkenoxids pro mol Mercaptan ist, M ein Alkalimetallatom und y die Molzahl der Alkalibase pro mol Mercaptan bezeichnet.

3. Verfahren nach Anspruch 1 oder 2, dadurch gekennzeichnet, daß x 1 bis 20, vorzugsweise 4 bis 12 ist, während y 0,01 bis 1, vorzugsweise 0,1 bis 0,5 ist.

4. Verfahren nach einem der vorhergehenden Ansprüche, dadurch gekennzeichnet, daß R ein $C_1$- bis $C_{20}$-, insbesondere ein $C_6$- bis $C_{18}$-Alkyl ist und das Alkenoxid $C_2$ bis $C_4$ und vorzugsweise Ethylenoxid ist.

5. Verfahren nach Anspruch 4, dadurch gekennzeichnet, daß das Alkyl verzweigt, insbesondere ein tertiäres Alkyl, und in ganz besonderer Weise tert.-Nonyl oder tert.- Dodecyl ist.

6. Verfahren nach einem der vorhergehenden Ansprüche, dadurch gekennzeichnet, daß das Erhitzen bei einer Temperatur von 60°C bis 150°C, insbesondere zwischen 70°C und 140°C stattfindet.

7. Verfahren nach Anspruch 6, in dem n einen Wert von 3 bis 5 hat, dadurch gekennzeichnet, daß man bei einer umso höheren Temperatur arbeitet je niedriger die gewünschte Zahl n ist.

8. Verfahren nach einem der vorhergehenden Ansprüche, dadurch gekennzeichnet, daß das Erhitzen des Mercaptans mit Schwefel und dem Katalysator in zwei Stufen durchgeführt wird und auf jede eine $H_2S$-Entgasung, insbesondere mittels eines Inertgases, folgt.

9. Verfahren nach einem der vorhergehenden Ansprüche, dadurch gekennzeichnet, das es unter einem relativen Druck von 0,2 bis 1 bar durchgeführt wird.

10. Verfahren nach einem der vorhergehenden Ansprüche, dadurch gekennzeichnet, daß der Katalysator durch Homogenisieren von einem mol Mercaptan mit $\alpha$y mol ($\alpha$ = 1 bis 1,5) Alkalibase, vorzugsweise zwischen 50°C und 100°C, hergestellt wird, danach das erhaltene Produkt mit Alkenoxid bei einer Temperatur von 80°C bis 120°C unter einem relativen Druck von 0,2 bis 4 bar gesättigt wird und anschließend eine Entgasung des überschüssigen Alkenoxids, vorzugsweise mittels eines Inertgases, erfolgt.

11. Verfahren nach Anspruch 10, in dem das Ausgangsmercaptan ein $C_6$ bis $C_{18}$-, insbesondere ein tertiäres Alkylmercaptan ist, dadurch gekennzeichnet, daß das Homogenisieren 4 bis 6 Stunden lang zwischen etwa 75°C und 85°C und die Sättigung mit Alkenoxid bei etwa 100 bis 110°C stattfindet.

12. Verfahren nach einem der vorhergehenden Ansprüche, dadurch gekennzeichnet, daß es kontinuierlich durchgeführt wird.

13. Neues katalytisches System, dadurch gekennzeichnet, daß es eine Zusammensetzung $R\text{-}S(C_mH_{2m}O)xH.(MOH)_y$ umfaßt, in der R ein Alkyl mit $C_1$ bis $C_{20}$, x eine Zahl von 1 bis 20, m eine Zahl von 2 bis 4, M ein Alkalimetall und y eine Zahl von 0,01 bis 1 ist.

14. Katalytisches System nach Anspruch 13, in dem R eine Alkylgruppe mit $C_6$ bis $C_{18}$, m gleich 2, x eine Zahl von 4 bis 12 und y eine Zahl von 0,1 bis 0,5 ist.

## Claims

1. Process for the production of organic polysulphides of the type $R\text{-}S_n\text{-}R$, where R is a hydrocarbon group and n a number from 2 to 8, by heating one or more mercaptans with sulphur, in the presence of a catalyst, characterized in that the catalyst consists of the combination of a mercaptan with an alkene oxide and an alkaline base.

2. Process according to Claim 1, characterized in that the catalyst is a combination $RSH.x(C_mH_{2m}O).yMOH$ where R denotes a hydrocarbon group, in particular an alkyl, x is the number of moles of alkene oxide per mole of mercaptan, M denotes an alkali metal atom and y the number of moles of alkaline base per mole of mercaptan.

3. Process according to Claim 1 or 2, characterized in that x is 1 to 20, and preferably 4 to 12, while y is 0.01 to 1, preferably 0.1 to 0.5.

4. Process according to one of the preceding claims, characterized in that R is a $C_1$-$C_{20}$, and in particular $C_6$-$C_{18}$, alkyl, the alkene oxide being $C_2$-$C_4$ and preferably ethylene oxide.

5. Process according to Claim 4, characterized in that the alkyl is branched, especially a tertiary alkyl, and in particular tert-nonyl or tert-dodecyl.

6. Process according to one of the preceding claims, characterized in that the heating takes place at a temperature of 60° to 150°C, more particularly between 70° and 140°C.

7. Process according to Claim 6, in which n has a value of 3 to 5, characterized in that the operation is carried out at a temperature which is proportionally higher the lower the desired number n.

8. Process according to one of the preceding claims, characterized in that the heating of the mercaptan with sulphur and the catalyst is carried out in two stages, each being followed by a degassing of $H_2S$, especially by means of an inert gas.

9. Process according to one of the preceding claims, characterized in that it is carried out under a relative pressure of 0.2 to 1 bar.

10. Process according to one of the preceding claims, characterized in that the catalyst is prepared by homogenization of one mole of a mercaptan with ay moles (a = 1 to 1.5) of alkaline base, preferably between 50° and 100°C, and then saturation of the product obtained with the alkene oxide at a temperature of 80° to 120°C under a relative pressure of 0.2 to 4 bars, followed by a degassing of the excess alkene oxide, preferably by means of an inert gas.

11. Process according to Claim 10, in which the starting mercaptan is $C_6$-$C_{18}$, in particular a tert-alkyl mercaptan, characterized in that the homogenization takes place between approximately 75° and 85°C for 4 to 6 hours and the saturation with the alkene oxide at approximately 100 to 110°C.

12. Process according to one of the preceding claims, carried out continuously.

13. New catalyst system characterized in that it comprises a combination $R\text{-}S(C_mH_{2m}O)_xH(MOH)_y$ in which R is a $C_1$-$C_{20}$ alkyl, x a number from 1 to 20, m a number from 2 to 4, M is an alkali metal and y a number from 0.01 to 1.

14. Catalyst system according to Claim 13, in which R is a $C_6$-$C_{18}$ alkyl group, m is equal to 2 and x is a number from 4 to 12 and y a number from 0.1 to 0.5.